# EUROPÄISCHE PATENTSCHRIFT

(11) **EP 0 271 715 B1**
(45) Veröffentlichungstag und Bekanntmachung des Hinweises auf die Patenterteilung: **27.01.1993**
(21) Anmeldenummer: 87116703.7
(22) Anmeldetag: 12.11.1987
(51) Int. Cl.: A61M 19/00, A61M 25/00, A61B 17/34

(54) **Stahlkanüle für die Spinal- und Periduralanästhesie**
Steel cannula for spinal and peridural anesthesia
Canule en acier pour l'anesthésie spinale et péridurale

(30) Priorität: 18.12.1986 DE 3643235
(43) Veröffentlichungstag der Anmeldung: 22.06.1988
(73) Patentinhaber: Pajunk, Horst, 78187 Geisingen (DE); Pajunk, Heinrich, 78187 Geisingen (DE)
(72) Erfinder: Witt, Hans-Hinrich, Dr., D-3501 Körle (DE)
(74) Vertreter: Patentanwälte Westphal, Buchner, Mussgnug Neunert, Göhring

(56) Entgegenhaltungen:
- EP-A- 0 092 389
- EP-A- 0 166 212
- DE-A- 3 020 926
- DE-A- 3 218 242
- DE-U- 8 236 443

## Beschreibung

Die Erfindung betrifft eine Stahlkanüle für die Spinal- und Periduralanästhesie nach dem Oberbegriff des Patentanspruchs 1.

Werden in der Spinal- oder Periduralanästhesie Kanülen mit schneidendem oder reißendem Schliff benutzt, so besteht die Gefahr von Gefäß-, Dura- und Nervenverletzungen. Insbesondere können Blutungen im Periduralraum, postspinale Kopfschmerzen oder vorübergehende bis persistierende neuronale Ausfälle auftreten.

Eine aus DE-A-30 20 926 bekannte Stahlkanüle, von der der Oberbegriff des Patentanspruchs 1 ausgeht, besteht aus einem geradlinigen Rohr, das am vorderen Ende als Spitze ausgebildet ist. Unmittelbar hinter dem Spitzenteil ist eine seitliche Öffnung in die Kanülenwand eingeschliffen. Diese langgestreckte Öffnung reicht bis nahezu zur Mittelachse der Kanüle und geht an ihrem vorderen und hinteren Rand kantenlos in den Spitzenteil bzw. die äußere Mantelfläche der Kanüle über. Der Rohrkanal erstreckt sich über das vordere Ende der Öfnnung hinaus bis in den Spitzenteil hinein. Eine derartige Stahlkanüle verursacht zwar ein kleines atraumatisches Punktionsloch, indem die Spitze die Längsfasern der Dura zerteilt, ohne diese Fasern zu zerschneiden, jedoch eignet sie sich nicht zum Plazieren eines Katheters sondern nur für die direkte Injektion von Anästhetikum durch den Kanülenkanal. Ein flexibler Katheter hat gegenüber der starren Stahlkanüle den Vorteil, daß er für die Injektion von Anästhetika und Medikamenten länger benutzt werden kann und auch nach dem Entfernen der zu seiner Verlegung benutzten Kanüle noch im Körper verbleiben kann.

Bekannt ist ferner eine Doppelkanüle (DE-OS 32 18 242) mit scharfer offener Spitze und einer Innenkanüle, deren stumpfe Spitze geschlossen und abgerundet ist. Seitlich hinter der stumpfen Spitze befindet sich eine Öffnung, aus der ein Katheter, der durch die Innenkanüle geschoben wird, unter Führung durch eine schräge Führungsfläche austreten kann. Nachteilig ist bei dieser Doppelkanüle der große Aufwand von Stichkanüle und Innenkanüle, die komplizierte Verlegungstechnik des Katheters, sowie die durch die stumpfe Spitze hervorgerufenen massiven Blutungen im Epiduralraum.

Aus der EP-A-0 166 212 ist eine Kunststoffkanüle bekannt, bei welcher ein Lappen aus der Kanülenwand thermoplastisch nach innen in den Rohrkanal geformt ist. Dadurch wird eine Führungsfläche gebildet, um das vordere Ende eines in die Kunststoffkanüle eingeschobenen Katheters seitlich aus der Kanüle austreten zu lassen und zu plazieren. Die Kunststoffkanüle eignet sich nicht für die Spinal- und Periduralanästhesie, da ihre Spitze offen und ihr Material zu weich ist. Zum Einführen der Kunststoffkanüle in den Körper des Patienten muß zunächst mittels einer Stahlstichkanüle ein Führungsmandrin in den Körper eingeführt werden, über welchen dann die Kunststoffkanüle geschoben werden kann. Die Kunststoffkanüle eignet sich daher nur dazu, Katheter in innere Weichorgane, wie z. B. die Leber, zu legen.

Der Erfindung liegt die Aufgabe zugrunde, eine Stahlkanüle nach dem Oberbegriff des Patentanspruchs 1, also eine einstückige, in einem Zug zu verlegende Kanüle, zu schaffen, die eine sichere Plazierung eines Katheters ermöglicht.

Die Lösung dieser Aufgabe erfolgt erfindungsgemäß mit den im kennzeichnenden Teil des Patentanspruchs 1 angegebenen Merkmalen.

Bei der erfindungsgemäßen Stahlkanüle, die mit ihrer atraumatisch spitz zulaufenden Spitze unmittelbar in die Dura eingestochen werden kann, ohne deren Fasern zu verletzen, ist sowohl eine direkte Inkjektion von Flüssigkeiten als auch die Verlegung eines Katheters möglich. Bei der Katheterverlegung wird der Katheter in dem Rohrkanal vorgeschoben. Wenn das vordere Katheterende die Führungsfläche erreicht, wird es von dieser Führungsfläche schräg nach vorne seitlich abgelenkt, so daß der Katheter präzise geführt plaziert werden kann. Dabei kann der Katheter insbesondere auch wieder in die Kanüle zurückgezogen werden, falls dies notwendig wird, ohne daß die Gefahr besteht, daß der Katheter durch die Kanüle beschädigt oder abgeschnitten wird. Da die Katheterspitze nicht traumatisch stumpf ist, verursacht ihr Vorschieben in den Periduralraum für den Patienten keine Schmerzen. Die atraumatische Spitze durchdringt sowohl Gewebe als auch die Nervenhaut ohne schneidend zu wirken oder eine wesentliche Stoßwirkung auszuüben. Das zu durchdringende Material wird vielmehr behutsam auseinandergeschoben und schließt sich nach dem Entfernen der Stahlkanüle wieder. Die Stahlkanüle ist für single-shot Applikation bestimmt, d. h. sie wird in einem Zug bis in den Periduralraum vorgeschoben.

Die Ausbildung der Führungsfläche als rückwärtige Stirnwand eines das vordere Ende des Rohrkanals bis an die Öffnung ausfüllenden Stopfens vereinfacht die Herstellung der Stahlkanüle und ermöglicht eine exakte Ausbildung der Führungsfläche, die eine glatte störungsfreie Führung der Katheterspitze gewährleistet.

Die seitliche Öffnung der Stahlkanüle ist nicht bis in den Bereich der Mittelachse der Kanüle eingeschliffen, obwohl die Breite der langgestreckten Öffnung etwa gleich dem Durchmesser des Rohrkanals ist. Die die Öffnung umgebende geschliffene Fläche bildet einen länglichen Trichter mit allseitig zum Öffnungsrand abfallender Trichterwand. Dadurch wird erreicht, daß die Außenkontur der Stahlkanüle im Bereich der Öffnung möglichst wenig verändert ist und daß der Rand der Öffnung an deren vorderem Ende ohne scharfe Kante in die Führungsfläche übergeht und beim gegenseitigen Verschieben von Katheter und Stahlkanüle keine schneidende Wirkung auf den Katheter ausüben kann.

Im folgenden wird unter Bezugnahme auf die Zeichnungen ein Ausführungsbeispiel der Erfindung näher erläutert.

Es zeigen:
- Fig. 1: einen Längsschnitt durch die Stahlkanüle,
- Fig. 2: einen Querschnitt entlang der Linie II-II von Fig. 1,
- Fig. 3: eine Draufsicht der Stahlkanüle und
- Fig. 4: eine Seitenansicht der Stahlkanüle beim Einführen eines Katheters.

Die dargestellte Stahlkanüle besteht aus einem langgestreckten geraden Rohr 10, das an seinem vorderen Ende mit einer einstückig angeformten rotationssymetrischen Spitze 11 verschlossen ist. Die Länge der Spitze 11 ist mindestens doppelt so groß wie der Außendurchmesser des Rohres 10, und insbesondere etwa 2,5-mal so groß. Die Außenfläche der rotationssymetrischen Spitze 11 ist leicht ballig, wobei der Übergang zur Außenfläche des Rohres 10 kontinuierlich, d.h. knickfrei, erfolgt.

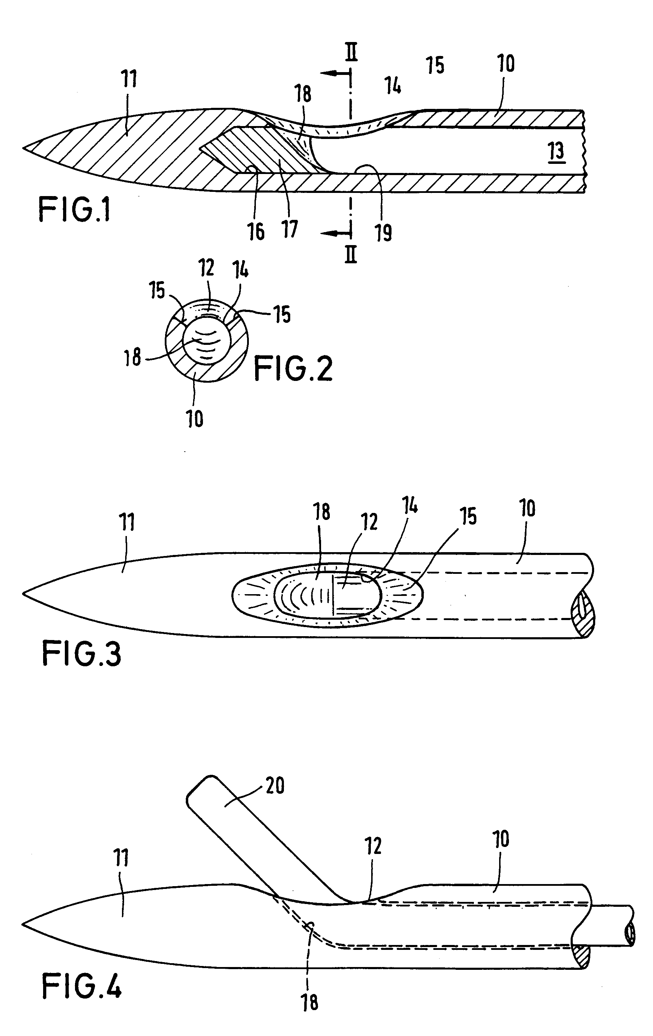

An dem Rohr 10 ist mit geringem Abstand hinter der Spitze 11 eine langgestreckte seitliche Öffnung 12 angebracht, die die Form einer Ellipse hat, deren Hauptachse parallel zur Rohrachse verläuft. Die Öffnung 12 steht mit dem Rohrkanal 13 in unmittelbarer Verbindung und sie wird von dem Rand 14 begrenzt. Der Rand 14 bildet gleichzeitig die innere Begrenzung einer geschliffenen Fläche 15, die die Öffnung 12 umlaufend umgibt. Die Fläche 15 ist eine allseitig zum Rand 14 abfallende Trichterwand. In jedem Querschnitt des Rohres verlaufen die beiden sich gegenüberliegenden Bereiche der Fläche 15 unter einem Winkel zueinander (Fig. 2), d.h. die sich gegenüberliegenden Flächenbereiche 15 verlaufen nicht in einer gemeinsamen Ebene. Dadurch wird erreicht, daß der äußere Rand der geschliffenen Fläche 15 an jeder Stelle höher liegt als der Rand 14 der Öffnung 12. Während der Rand 14 bis relativ nahe an die parallel zur Öffnung 14 verlaufende Längsmittelebene des Rohres 10 heranreicht, hat der äußere Rand der Fläche 15 einen größeren Abstand von dieser Ebene.

Der Rohrkanal 13 ist durch einen über das vordere Ende der Öffnung 12 hinausragenden Fortsatz 16 verlängert. Dieser Fortsatz 16 ist mit einem Stopfen 17 aus Lötmaterial, Kunststoff, o.dgl. ausgefüllt, so daß der lichte Rohrkanal an dem Stopfen 17 endet. Das rückwärtige Ende des Stopfens 17 ist als Führungsfläche 18 ausgebildet, die vom Boden 19 des Rohrkanals 13, d.h. von derjenigen Innenfläche, die der Öffnung 12 abgewandt ist, stetig nach vorne und seitlich in Richtung auf den Rand 14 ansteigt. Die Führungsfläche 18 ist - bezogen auf den Rohrkanal - konvex geformt, d.h. in Richtung auf den Stopfen 17 durchgebogen. Außerdem ist die Führungsfläche 18 in Querrichtung des Rohres 10 gebogen, und zwar entsprechend dem Verlauf des Randes 14 am vorderen Ende der Öffnung 12. Das obere Ende der Führungsfläche 18 schließt sich unmittelbar an den Rand 14 an. Die Führungsfläche dient dazu, einen Katheter 20, der durch das Rohr 10 hindurch vorgeschoben wird, seitlich aus der Öffnung 12 herauszuführen, so daß das Katheterende schräg nach vorne vom Rohr 10 absteht, wie es in Fig. 4 dargestellt ist. Das Rohr 10 kann in dieser Position zurückgezogen werden, während der Katheter 20 in seiner Stellung verbleibt und von der Führungsfläche 18 seitlich aus dem Rohr herausgedrückt wird.

Der Katheter 20 besteht in bekannter Weise aus einem flexiblen Schlauch. Sein Durchmesser ist kleiner als der Durchmesser des Rohrkanals 13, so daß der Katheter im Rohrkanal leicht vorgeschoben werden kann. Da der Durchmesser der Öffnung 14 in Querrichtung nur geringfügig größer ist als der Durchmesser des Rohrkanals 13, kann der Katheter 20 die Öffnung 14 leicht passieren. Am rückwärtigen Ende der langgestreckten Öffnung 12 sollte der Rand 14 möglichst stumpf sein, damit er beim Zurückziehen des Katheters 20 nicht in diesen einschneidet.

Die Länge der Öffnung 14 beträgt maximal das Doppelte der Breite dieser Öffnung, so daß der Katheter eng anliegt und steil in Richtung der Führungsfläche 18 aus der Stahlkanüle herausgeführt wird.

## Patentansprüche

1. Stahlkanüle für die Spinal- und Periduralanästhesie, bestehend aus einem geraden Rohr (10), das am vorderen Ende eine atraumatisch spitz zulaufende rotationssymmetrische Spitze (11) und hinter der Spitze (11) eine längliche, seitliche Öffnung (12) des Rohrkanals (13 aufweist, wobei der Durchmesser der Öffnung (12) in Querrichtung annähernd gleich dem Durchmesser des Rohrkanals (13) ist, dadurch gekennzeichnet, daß der Rohrkanal (13) an einer Führungsfläche (18) endet, die vom Boden (19) des Rohrkanals (13) schräg zum vorderen Ende der Öffnung (12) ansteigt und an der rückwärtigen Stirnwand eines Stopfens (17) ausgebildet ist, der eine einen Fortsatz (16) des Rohrkanals (13) bildende Sackbohrung ausfüllt, derart daß ein im Rohrkanal (13) verschiebbarer Katheter (20) unit dessen vorderem Ende unter Führung durch die Führungsfläche (18) seitlich aus dem Rohr (10) austreten kann.

2. Stahlkanüle nach Anspruch 1, dadurch gekennzeichnet, daß die Führungsfläche (18) parallel zum Rand (14) des vorderen Endes der Öffnung (12) gebogen ist.

## Claims

1. A steel cannula for spinal and peridural anaesthesia, comprising a straight tube (10) having at the front end an axially symmetrical tip (11) converging to an atraumatic point and rearwardly of the tip (11) an elongate lateral opening (12) of the tube channel (13), the diameter of the opening (12) in the transverse direction being substantially equal to the diameter of the tube channel (13), characterized in that the tube channel (13) terminates at a guide face which rises from the bottom (19) of the tube channel (13) at an inclination in the direction of the front end of the opening (12) and is formed on the rearward end wall of a plug (17) which so fills a blind bore forming an extension (16) of the tube channel (13) that a catheter (20) slidable in the tube channel (13) can emerge by its front end laterally from the tube (10) while being guided by the guide face (18).

2. A steel cannula according to claim 1, characterized in that the guide face (18) is bent parallel with the edge (14) of the front end of the opening (12).

## Revendications

1. Canule d'acier pour l'anesthésie spinale ou péridurale, constituée d'un tube droit (10) qui présente à l'extrémité antérieure une pointe (11) à symétrie de rotation s'étendant de façon atraumatique et en arrière de la pointe (11) se trouve un orifice (12) latéral longitudinal du canal tubulaire (13), le diamètre de l'orifice (12) en sens transversal est presque identique au diamètre du canal tubulaire (13), caractérisée en ce que le canal tubulaire (13) se termine par une surface de guidage qui monte du fond (19) du canal tubulaire (13) obliquement vers l'extrémité antérieure de l'orifice (12) et sur la face arrière d'un bouchon (17), qui remplit un prolongement (16) du canal tubulaire (13), de sorte qu'un cathéter (20) pouvant coulisser dans le canal tubulaire (13) peut sortir latéralement du tube (10) avec son extrémité antérieure par guidage par la surface de guidage (18).

2. Canule en acier selon la revendication 1, caractérisée en ce que la surface de guidage (18) est incurvée parallèlement au bord (14) de l'extrémité antérieure de l'orifice (12).
